Europäisches Patentamt

European Patent Office

Office européen des brevets

(19)

(11) Publication number: **0 256 159 B1**

(12) **EUROPEAN PATENT SPECIFICATION**

(45) Date of publication of patent specification: **29.05.91**

(51) Int. Cl.⁵: **A61B 5/022**, A61B 5/0402, A61B 5/0245

(21) Application number: **86111425.4**

(22) Date of filing: **19.08.86**

(54) Method and system for monitoring blood pressure.

(43) Date of publication of application:
24.02.88 Bulletin 88/08

(45) Publication of the grant of the patent:
29.05.91 Bulletin 91/22

(84) Designated Contracting States:
DE FR GB

(56) References cited:
EP-A- 0 182 197
US-A- 4 245 648
US-A- 4 299 234
US-A- 4 396 018
US-A- 4 617 937

MEDICAL AND BIOLOGICAL ENGINEERING &
COMPUTING, vol. 19, no. 5, September 1981,
pages 671, 672,Stevenage, GB; L.A. Geddes
et al.: "Pulse arrival time as a method of
obtaining systolic and diastolic blood pres-
sure indirectly"

(73) Proprietor: COLIN ELECTRONICS CO., LTD.
2007-1, Hayashi
Komaki-shi Aichi-ken(JP)

(72) Inventor: Peel, Harry Herbert
7535 Pipers Creek
San Antonio Texas 78251(US)
Inventor: Brisco, John Steven
3908 Warbow
San Antonio Texas 78238(US)
Inventor: Moczygemba, Mark Edward
7108 Cloudview
San Antonio Texas 78250(US)
Inventor: Neatherlin, Robert Louis
8326 Dragon
San Antonio Texas 78250(US)
Inventor: Sargent, Brenda Ann
4019 Belle Grove
San Antonio Texas 78230(US)

(74) Representative: Grupe, Peter, Dipl.-Ing. et al
Patentanwaltsbüro Tiedtke-
Bühling-Kinne-Grupe-Pellmann-Grams-Struif
Bavariaring 4
W-8000 München 2(DE)

## Description

This invention relates to blood pressure measurement in general and in particular to automatic blood pressure monitoring systems. Still more particularly, this invention relates to a method and apparatus for accurately monitoring blood pressure during stress testing or other physical activity as well as when quiescent.

One of the major medical problems facing the public today is associated with the lack of control of abnormal blood pressure. For control of abnormal blood pressure, accurate and dependable measurement of blood pressure is necessary both to determine the presence of a problem and for monitoring the pressure to assure alleviation or control of such problem. In particular, it is highly useful for the medical practitioner to determine a patient's blood pressure during physical activity such as during a diagnostic stress test; however, such testing generally results in severe and frequent arm motions which generate a large number of false signals known as motion artifacts.

As a result of the ploblems generated by these motion artifacts, numerous attempts have been made to provide an apparatus for measuring systolic and diastolic blood pressure during such testing. However, since motion artifacts have frequency similar to that of Korotkov sounds, it is difficult to distinguish motion artifacts from Korotkov sounds. Therefore, such an apparatus has a problem that, when motion artifacts are eliminated, some Korotkov sounds are eliminated at the same time. One of such blood pressure monitoring systems is disclosed in United States Patent No. 4,408,614, issued to Charles S. Weaver et al. Weaver et al provides an automatic computer-implemented technique for identifying and eliminating false outputs from a Korotkov sound detector included in a measuring system or the like which is adapted for use during stress testing.

US-A-4396018 discloses a blood pressure monitoring system, wherein sound pulses proximal and distal an inflatable cuff are detected.

Known systems do generally provide some degree of enhanced accuracy due to elimination of motion artifacts, but they are not satisfactory. In blood pressure measurement for medical diagnoses, some patients may rapidly move their arms from a state of rest and other patients will undergo a stress test requiring their hard physical activity. If any motion artifacts produced in such blood pressure monitoring are eliminated by the system, Korotkov sounds utilized to determine blood pressure may not be satisfactorily obtained. Consequently, the blood pressure measurement may not be conducted accurately or smoothly. It is therefore accepted that an improved blood pressure monitoring system which may be utilized during diagnostic stress testing or other physical activity would be highly desired.

The inventors have earnestly studied in the above described background, and found a fact that the curve representing the relation between the products resulting from multiplying distal pulse sound amplitudes by the time delays from proximal pulse sound to distal pulse sound, and the pressure cuffs, has large changings at points which correspond to the systolic and diastolic pressure. The concept of the present invention is based on the above fact.

According to the first aspect of the present invention, there is provided a method of obtaining accurate blood pressure measurement by utilizing an inflatable cuff applied to a part of a subject's body, comprising the steps of: (a) detecting a pulse sound at a proximal position of the inflatable cuff; (b) detecting the same pulse sound at a distal position of the inflatable cuff; (c) measuring an amplitude of the pulse sound detected at the distal position of the inflatable cuff; (d) measuring a cuff pressure of the inflatable cuff at the time of detecting the pulse sound at the distal position of the inflatable cuff; (e) measuring a time delay between each pulse sound at the proximal position of the inflatable cuff and the same pulse sound at the distal position of the inflatable cuff; (f) multiplying the amplitude of the pulse sound at the distal position of the inflatable cuff by the time delay; and (g) determining a measure of systolic and/or diastolic blood pressure of the subject's body based on the relation between the resultant products and the cuff pressures measured.

According to the second aspect of the invention, there is provided a blood pressure monitoring system for measuring blood pressure by utilizing an inflatable cuff applied to a part of a subject's body, comprising: (1) proximal audio transducer means for detecting a pulse sound at a proximal position of the inflatable cuff; (2) distal audio transducer means for detecting the same pulse sound at a distal position of the inflatable cuff and for measuring an amplitude of the pulse sound detected; (3) pressure transducer means for measuring a cuff pressure of the inflatable cuff at the time of detecting the pulse sound at the distal position of the inflatable cuff; (4) time delay measurement means for obtaining a time delay between each pulse sound at the proximal position of the inflatable cuff and the pulse sound at the distal position of the inflatable cuff; (5) calculating means for multiplying the amplitude of the pulse sound at the distal position of the inflatable cuff by the time delay; and (6) blood pressure measurement means for obtaining a measure of systolic and/or diastolic blood pressure based on the relation between the resultant products and the cuff pressures measured.

In the above blood pressure monitoring method and system, a blood pressure evaluation curve is obtained by taking the relation between the distal pulse sound amplitudes and the transit time delays. The evaluation curve is utilized to determine the systolic and diastolic pressure. The evaluation curve has large changings at positions which correspond to the systolic and diastolic blood pressure. These changings on the evaluation curve are so sharp that the blood pressure is accurately determined in spite of possible minglings of noise.

In accordance with the third aspect of the invention, there is provided a blood pressure monitoring system for measuring blood pressure by utilizing an inflatable cuff applied to a part of a subject's body, comprising: (1) proximal audio transducer means for detecting a pulse sound at a proximal position of the inflatable cuff; (2) distal audio transducer means for detecting the same pulse sound at a distal position of the inflatable cuff and for measuring an amplitude of the pulse sound detected; (3) pressure transducer means for measuring a cuff pressure of the inflatable cuff at the time of detecting the pulse sound at the distal position of the inflatable cuff; (4) electrocardiographic lead means providing at least one electrocardiographic lead which provides an electrocardiographic signal from the subject's body; (5) gate means for permitting the pulse sound to pass therethrough for a predetermined period in response to the electrocardiographic signal; (6) time delay measurement means for obtaining a time delay between each pulse sound at the proximal position of the inflatable cuff and the pulse sound at the distal position of the inflatable cuff; (7) calculating means for multiplying the amplitude of the pulse sound at the distal position of the inflatable cuff by said time delay; and (8) blood pressure measurement means for obtaining a measure of systolic and/or diastolic blood pressure based on the relation between the resultant products and the cuff pressures measured.

The above system of the invention has a more advantageous anti-noise characteristic due to gate means for permitting pulse sounds to pass therethrough during a predetermined open length.

In the preceding blood pressure monitoring system, the electrocardiographic lead means may comprise a plurality of electrocardiographic electrodes which are attached to the subject's body, the plurality of electrocardiographic electrodes providing the at least one electrocardiographic lead.

Also, in the same blood pressure monitoring system, the at least one electrocardiographic lead may comprise a plurality of electrocardiographic leads and the electrocardiographic lead means may select a particular electrocardiographic lead from the plurality of electrocardiographic leads. In this form of blood pressure monitoring system, the electrocardiographic lead means may comprise means for periodically measuring the amount of noise present on each of the plurality of electrocardiographic leads, and also comprises means for selecting an electrocardiographic lead having the least amount of noise from the plurality of electrocardiographic leads. Further, in the same form of blood pressure monitoring system, the electrocardiographic lead means may comprise means for detecting the presence or absence of an electrocardiographic signal and loss of said signal from said particular electrocardiographic lead may cause the electrocardiographic lead means to utilize a second lead.

The novel features believed characteristic of the invention are set forth in the appended claims. The invention itself, however, as well as a preferred mode of use, further objects and advantages thereof, will best be understood by reference to the following detailed description of an illustrative embodiment when read in conjunction with the accompanying drawings, wherein:

Figure 1 depicts a diagrammatic view of a patient undergoing a diagnostic stress test utilizing the blood pressure monitoring system of the present invention;

Figure 2 depicts an enlarged view of the electronic unit of the blood pressure monitoring system of the present invention which illustrates the connections between the patient and the blood pressure monitoring system of the present invention;

Figure 3 depicts a block diagram of the blood pressure monitoring system of the present invention;

Figure 4A depicts a graph of pulse sound amplitudes plotted versus cuff pressures when utilizing the novel blood pressure monitoring system of the present invention;

Figure 4B depicts a graph of the time dalay between proximal pulse sound and distal pulse sound versus cuff pressure when utilizing the novel blood pressure monitoring system of the present invention;

Figure 4C depicts a graph of a blood pressure evaluation curve which is obtained by determining the product of the pulse amplitudes depicted in Figure 4A and the time delays depicted in Figure 4B versus cuff pressure with the novel blood pressure monitoring system of the present invention;

Figures 5, 5A, 5B, 5C, 5D, 5E and 5F depict a logic flow diagram for the operation of the novel blood pressure monitor system of the present invention, respectively;

Figures 6A and 6B depict a group of steps in block 170 of Figure 5B, respectively; and

Figures 7, and 8 and 9 depict an interrupt routine executed in Figures 5A, 5B and 5C, respectively.

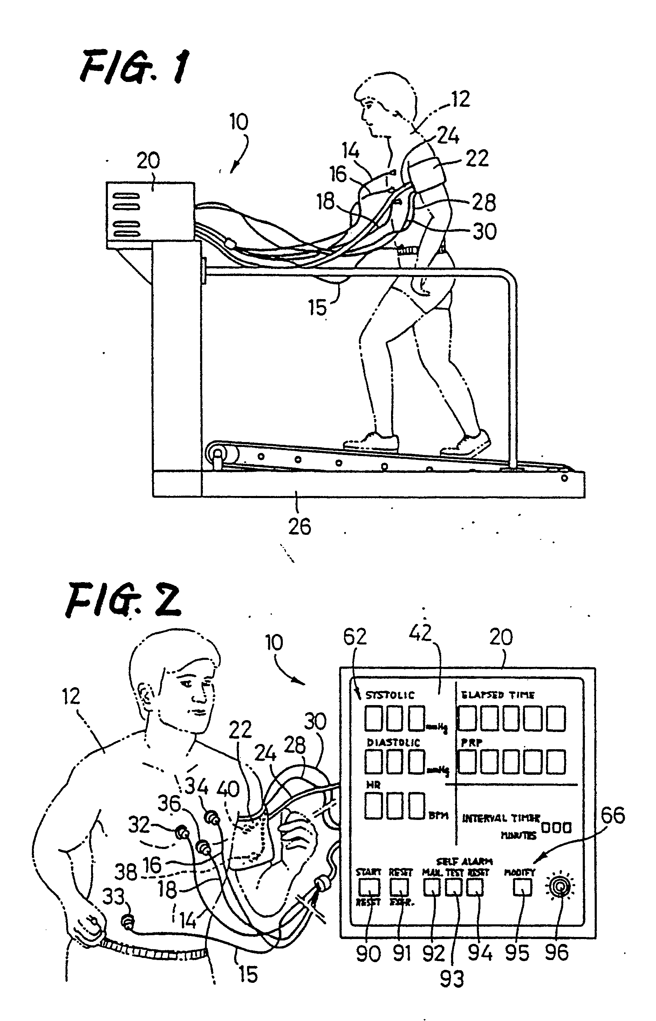

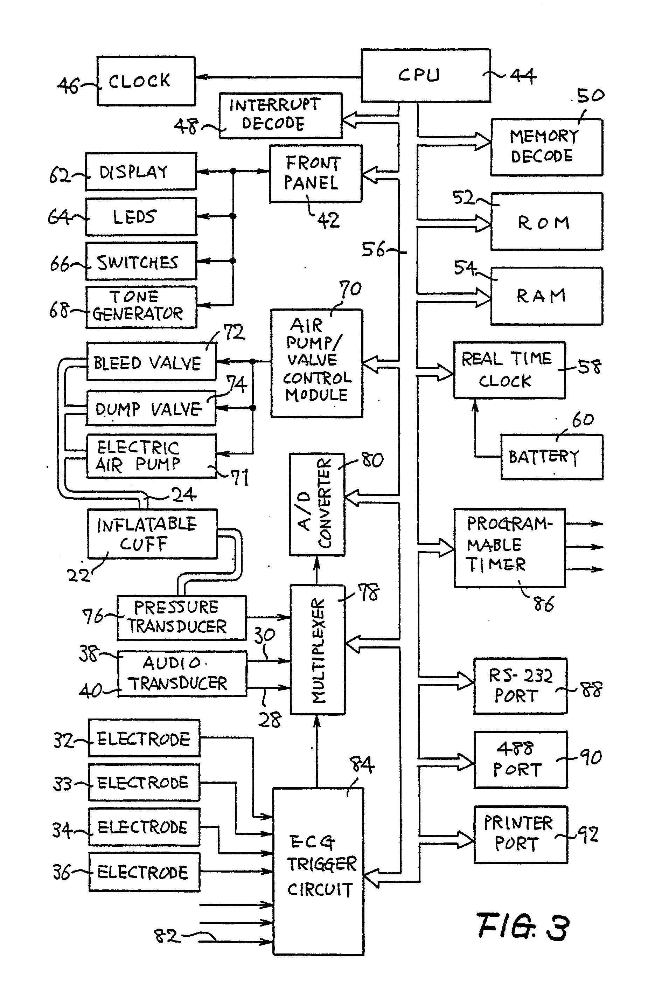

With reference now to the figures and in particular with reference to Fig. 1, there is depicted a diagrammatic view of a patient stress test utilizing blood pressure monitoring system 10 of the invention. As can be seen, blood pressure monitoring system 10 includes an electronic unit 20 which is coupled to patient or subject 12 utilizing a plurality of electrocardiographic electrodes 32, 33, 34 and 36 (shown in Fig. 2) and which also includes an inflatable cuff 22. Flexible air tube 24 is coupled to inflatable cuff 22 and is utilized in conjunction with an electric air pump 71 (shown in Fig. 3) to inflate and deflate inflatable cuff 22 within a range of pressures within which Korotkov sounds are produced. As is typical in diagnostic stress testing, patient 12 is depicted walking on a treadmill 26 and in all probability, the patient's arms will be moving, generating so-called "artifact noise". The artifact noise will render accurate measurement of blood pressure highly difficult because it has substantially the same frequency characteristic as that of Korotkov sound. Concealed within inflatable cuff 22 are two audio transducers 38 and 40 (shown in Fig. 2) which are utilized to convert pulse sounds to electric signals. These signals are then coupled to electronic unit 20 by means of wires 28 and 30, as will be illustrated herein.

Referring now to Figure 2, there is depicted an enlarged view of electronic unit 20 of blood pressure monitoring system 10 of the present invention which clearly demonstrates the connections between patient 12 and blood pressure monitoring system 10. As may be seen clearly, four electrocardiographic electrodes 32, 33, 34 and 36 are coupled to electronic unit 20 by means of wires 14, 15, 16 and 18 and provide three electrocardiographic leads.

As will be explained in greater detail herein, electronic unit 20 then selects an optimum lead from among the three electrocardiographic leads in order to obtain a trigger signal therefrom. Disposed within inflatable cuff 22 are two audio transducers 40 and 38. Audio transducer 40 is referred to as the proximal audio transducer and is located near or proximate to the subject's body, along the proximal edge of inflatable cuff 22. Audio transducer 38 is referred to as the distal audio transducer and is located away from the subject's body along the distal edge of inflatable cuff 22. As can be seen, electronic unit 20 includes a front panel 42 upon which systolic and diastlic pressure as well as elapsed time, pulse rate and interval between blood pressure measurements may be displayed. Additionally, electronics unit 20 also includes a plurality of switches which permit the various modes of operation to be selected.

With reference new to Figure 3, there is depicted a simplified block diagram of blood pressure monitoring system 10 of the present invention. Where possible, the elements within the block diagram of Figure 3 have been given the same reference numerals as utilized in Figures 1 and 2. As can be seen, blood pressure monitoring system 10 utilizes a microprocessor 44 to control the operation of the system. In a preferred embodiment of the present invention, microprocessor 44 is an MC68000 microprocessor and is utilized in conjunction with clock 46 which provides an 8 MHz- (megaherz) clock output. Interrupt decode 48 and memory decode 50 are utilized in conjunction with microprocessor 44. Interrupt decode 48 includes interrupt decode logic and system reset logic. Memory decode 50 is utilized to permit data stored in memory to be addressed and buffered for access by microprocessor 44.

Microprocessor 44 is capable of addressing both read-only-momory 52 and random-access-memory 54. In a preferred embodiment of the present invention, 128 K bytes are provided in both read-only-memory 52 and random-access memory 54. A real-time clock 58 is provided and coupled to microprocessor 44 along bus 56. Real-time clock 58 is utilized to permit the actual time and date of a particlular test to be stored in memory with data resulting from that test. A battery 60 is also provided to ensure that real-time clock 58 does not need to be periodically reset should power to blood pressure monitoring system 10 be interrupted.

As disclosed in Figure 2, the front panel 42 of electronic unit 20 includes both displays 62, LED indicators 64 and switches 66 which permit the various modes of operation to be selected. Additionally, a tone generator 68 is provided and is utilized to generate an alarm tone under alarm conditions.

Air pump and valve control module 70 is utilized to control electric air pump 71, bleed valve (deflation control valve or slow bleed valve) 72 and dump valve (rapid dump valve) 74 and to inflate and deflate inflatable cuff 22. The air pump and valve control module 70, such as Model No. FN30-S4668 manufactured by Cannon Seiki of Japan, preferably controls two electric air pumps and bleed and dump valves. Bleed valve 72 and dump valve 74 are provided to permit the pressure within inflatable cuff 22 to be slowly bled off or rapidly dumped in the event that the process must begin again rapidly or the measurement has been completed.

Outputs from pressure transducer 76 and audio transducers 38 and 40 which represent the pressure within inflatable cuff 22 and the presence of pulse sounds detected by audio transducers 38 and 40 are coupled to multiplexer 78 which includes analog signal conditioning circuitry, channel decode logic and analog control logic. The output of multiplexer 78 is then passed to a sample and hold circuit of analog to digital converter 80 and then to a twelve bit analog to digital converting circuit of the same 80.

Electrocardiographic signals 82 from electrodes 32, 33, 34 and 36 or external electrocardiographic lead signals 82 may be coupled to electrocardiographic trigger circuit 84 which serves as electrocardiographic lead means of the present invention. Electrocardiographic trigger circuit 84 consists of electrocardiographic circuitry, floating lead detector circuitry and selector circuitry. The floating lead detector circuitry detects floating lead noise present on each of the three electrocardiographic leads. The selector circuitry selects an optimum lead from the three leads and couples it to the electrocardiographic circuitry. The electrocardiographic circuitry amplifies and modifies an optimum lead signal into a trigger signal and generates it. More specifically described, the floating lead detector circuitry periodically samples the floating lead noise which is associated with commercial frequency, such as 60 Hertz, and which increases when an electrode becomes detached from the patient. The selector circuitry is switched by microprocessor 44 to select the particular or optimum lead that has the least amount of floating lead noise as well as a satisfactory amplitude of electrocardiographic signal. In this way, the one electrocardiographic lead is selected from the three leads, which represents the best signal to generate a trigger signal. As a result, blood pressure monitoring system 10 will not fail should an electrocardiographic electrode become dislodged or should an unusual amount of noise be present on a particular electrode. Additionally, microprocessor 44 includes means for detecting the absence or presence of an electrocardiographic signal so that should an electrocardiographic signal be lost the selector circuitry of electrocardiographic trigger circuit 84 may be immediately switched to select another or second electrocardiographic lead to obtain a trigger signal and continue blood pressure measurement.

A real time clock 58 such as Model MC146818 integrated circuit timer is also provided to permit blood pressure monitoring system 10 to accurately and correctly determine intervals between successive blood pressure measurements. Finally, blood pressure monitoring system 10 also includes several output ports to permit data from blood pressure monitoring system 10 to be coupled to a variety of devices. Included among these output ports is RS-232 port 88 which preferably is implemented utilizing an MC6850 asynchronous communication interface adaptor, baud rate generator and various line drivers and receivers. The baud rate in this device is switch selectable from 50 to 19,200 baud and thus permits data from blood pressure monitoring system 10 to be transmitted serially to another device utilizing a similar port. Next, a general purpose interface circuit, i.e., 488 port 90 which utilizes a standard IEEE-488 connector is also provided to permit blood pressure monitoring system 10 to communicate with additional devices. Finally, a printer output port 92 is also provided so that blood pressure measurement data derived by blood pressure monitoring system 10 may be coupled to a printer to provide hard copy.

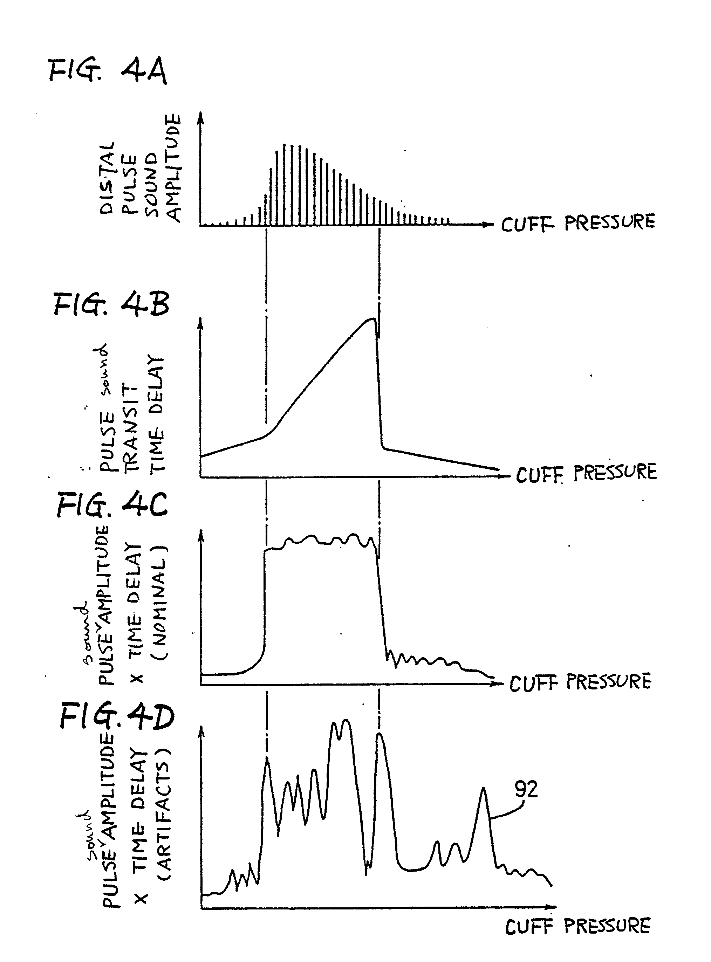

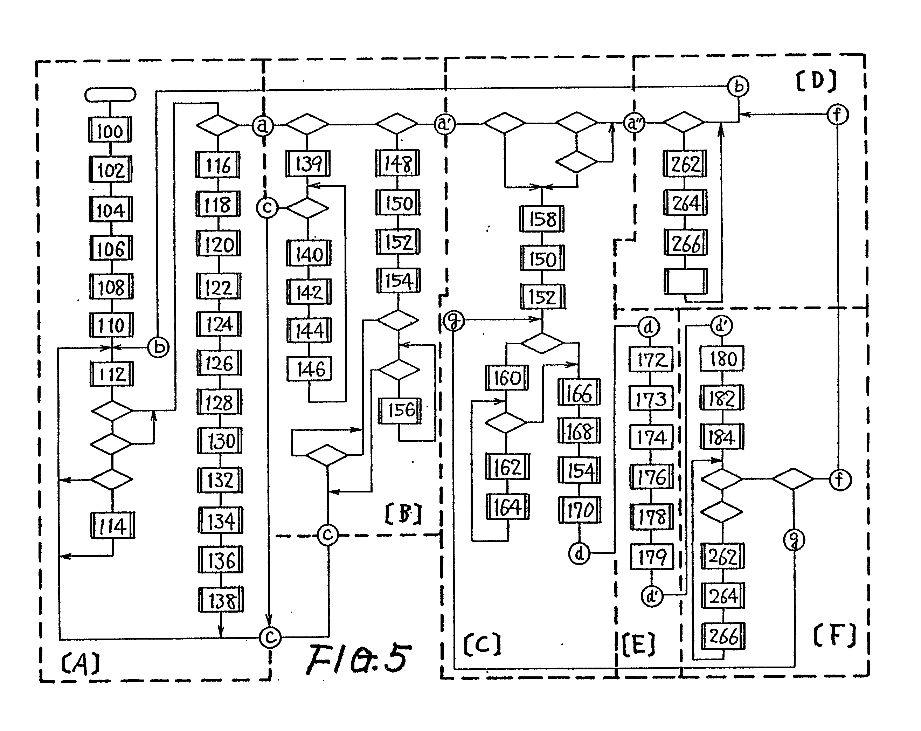

With reference now to Figure 4A there is depicted a graph of pulse sound amplitudes plotted versus cuff pressures. As can be seen, the amplitude of pulse sounds measured at distal audio transducer 38 will increase as the pressure within inflatable cuff 22 bleeds off. The point where the pulse sounds detected at distal audio transducer 38 become audible is generally referred to as the systolic blood pressure. It should be noted that as cuff pressure diminishes further from right to left in the graphs of Figure 4A the distal pulse sound amplitudes drop off rapidly at a point which is acknowledged to be the diastolic blood pressure. Many blood pressure monitoring systems utilizes distal pulse sound amplitudes to detect both systolic and diastolic blood pressures by determining the point at which the distal pulse sound amplitudes first exceed a given reference value and the point thereafer at which the distal pulse sound amplitudes fall below a given reference value. It should be noted that the values depicted in Figure 4A are ideal in nature and that in actual practice artifact noises make these two points substatially more difficult to locate.

Referring now to Figure 4B, there is depicted a graph of the time delay between a pulse sound detected at the proximal audio transducer 40 and the sound generated by the same pulse when it reaches the distal audio transducer 38. As can be seen, at high cuff pressures a small amplitude distal pulse sound is present which occurs at practically the same time as the pulse sound detected by the proximal audio transducer 40. At a point generally determined to be the systolic blood pressure the transit time or delay between a pulse detected at the proximal audio transducer 40 and the same sound detected at the distal audio transducer 38 becomes quite high. Thereafter, as cuff pressure continues to diminish the time delay between the moment a pluse is detected at the proximal audio transducer 40 and the moment that pulse is detected at the distal audio transducer 38 decreases generally linearly to a point where the decrease shifts slightly. That point is generally referred to as the diastolic blood pressure. Again, the graph depicted in Figure 4B is ideal in nature and the presence of artifact noise makes this method highly difficult to utilize to accurately determine systolic and diastolic blood pressure.

With reference now to Figure 4C, there is depicted a graph of a novel blood pressure evaluation curve which is utilized by blood pressure monitoring system 10 of the present invention. The graph in Figure 4C is obtained by multiplying the pulse amplitudes present in Figure 4A by the pulse transit time delay values depicted in graph 4B. As may be seen, the combination of these two measurements results in an evaluation curve which includes highly distinguishable transitions at the systolic and diastolic pressure points. The utilization of this evaluation curve greatly enhances the accuracy of blood pressure monitoring system 10 and permits systolic and diastolic pressure to be accurately determined by an evaluation curve.

Finally, Figure 4D depicts a graph of a blood pressure evaluation curve similar to that depicted in Figure 4C wherein there are large artifact errors due to physical activity as depicted in Figure 1. As may be seen, the graph in Figure 4D would make evaluation of systolic and diastolic transition points rather difficult. In order to successfully determine systolic and diastolic pressure points when utilizing a curve similar to that depicted in Figure 4D, an additional filter and analysis step is required to eliminate errors which may be caused by artifact noise. Furthermore, gate means (which will be described in detail) may be utilized to eliminate noise.

The analysis of the graph depicted in Figure 4D is accomplished by digital operation on the values depicted therein by means of microprocessor 44 (see Figure 3). In a preferred embodiment of the present invention each cuff pressure point on graph 4D (that is, product of distal pulse sound amplitude by transit time delay) is analyzed utilizing a so-called "median" filter. A median filter analyzes several successive points and substitutes the median value point for the middle point of the analyzed points. Five data points are used in this embodiment. In this manner, large transitions followed by a recession to nominal values will be eliminated, which provides a smoothed evaluation curve.

Next, a weighted average filter (which will be described in detail) may then be applied to achieve further smoothing of the curve which results from the utilization of the median filter.

Those ordinary skilled in the art will appreciate that while these two well known filtering techniques are utilized in the depicted embodiment of the present invention that other statistical smoothing techniques may be equally advantageous and may also be utilized.

Finally, a statistical analysis approach is utilized to analyze the resultant curve to determine the transitions points which represent systolic and diastolic pressure. Most known blood pressure monitoring systems utilize an absolute level which is predetermined and report systolic pressure at the first point which occurs above this nominal level and diastolic pressure at the first point thereafter which occurs below the predermined level. In contrast, blood pressure monitoring system 10 of the present invention utilizes a statistical analysis to determine whether or not a particular rise in the curve represents the systolic pressure.

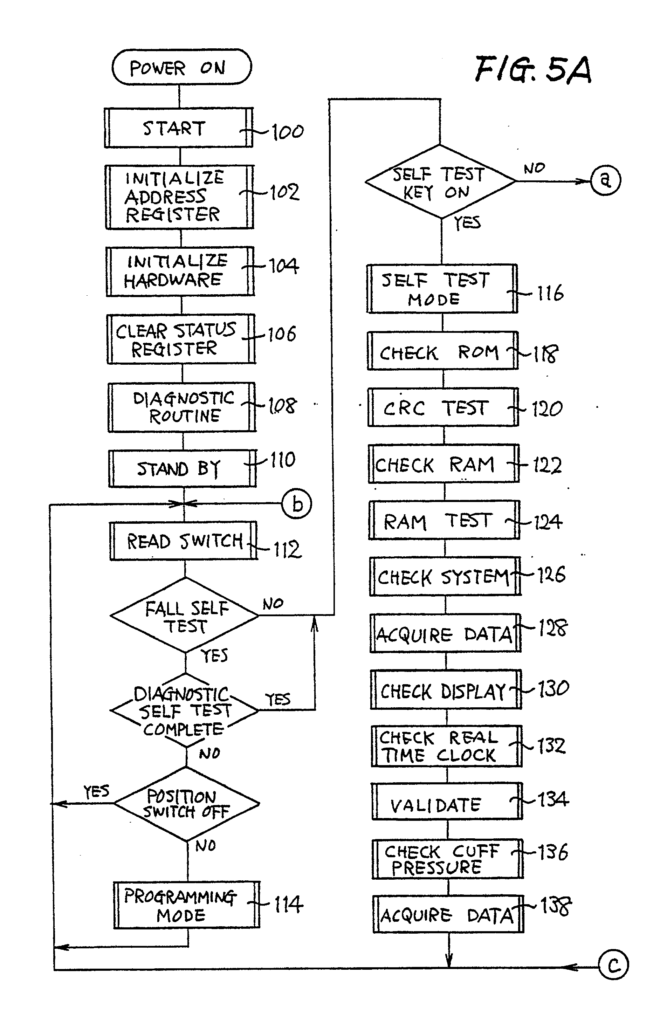

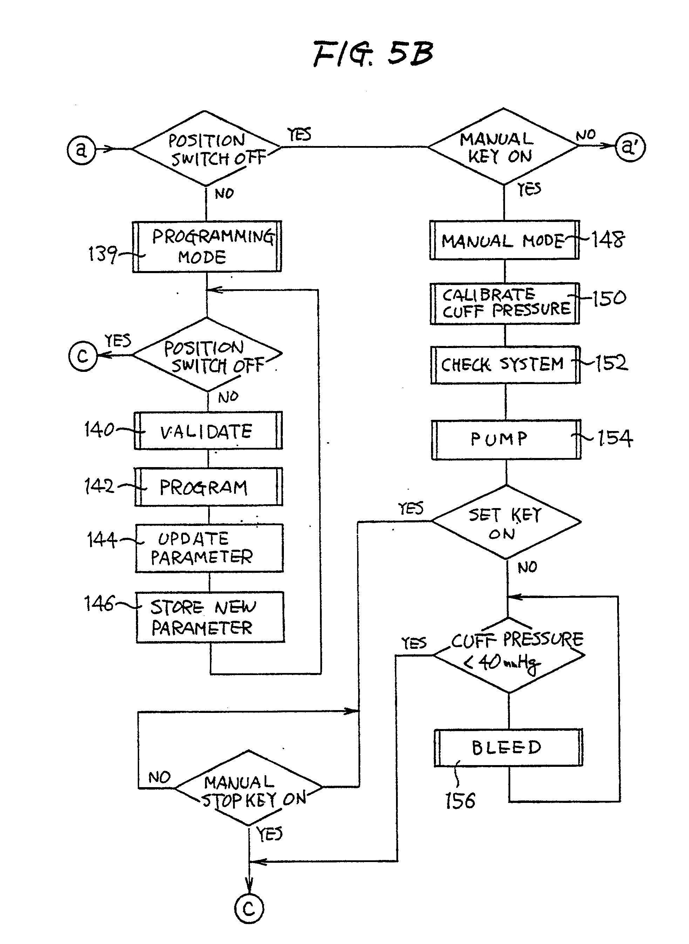

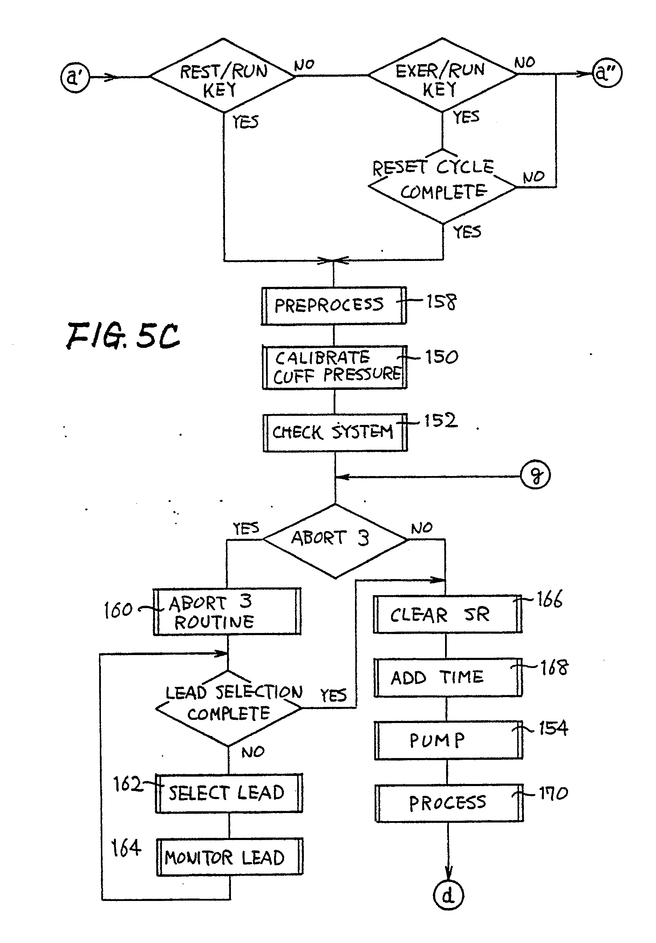

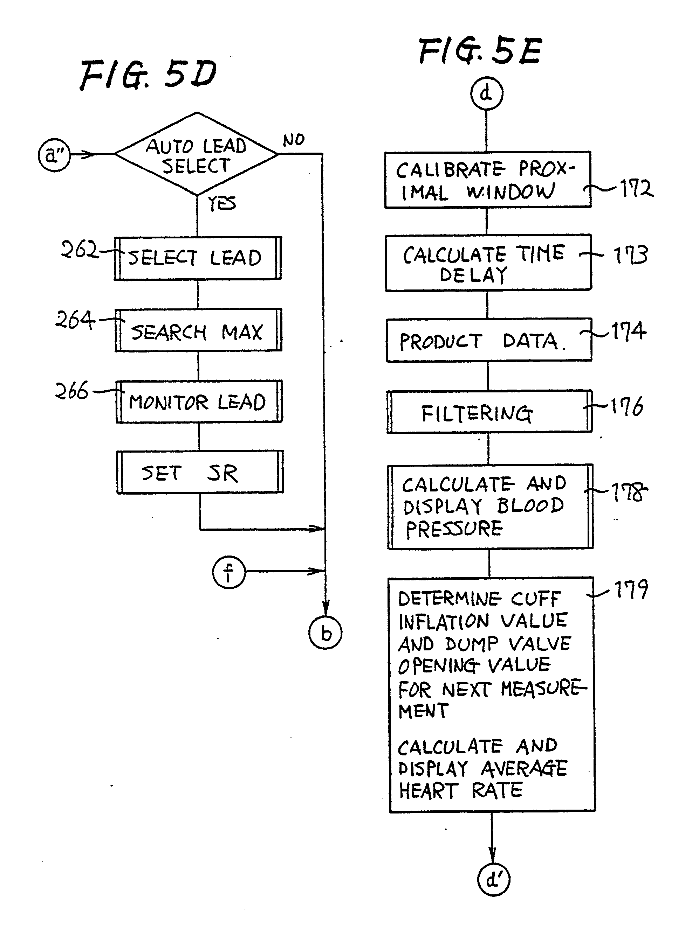

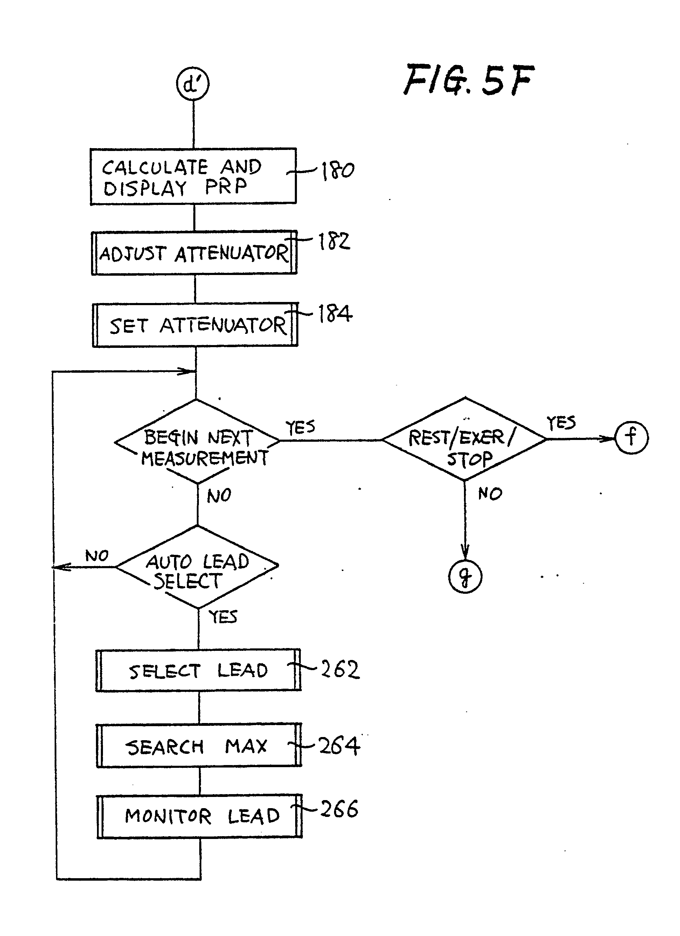

Now referring to Figures 5, 5A, 5B, 5C, 5D, 5E, and 5F there is depicted a logic flow diagram for the operation of blood pressure monitoring system 10 of the present invention.

Processing of blood pressure information is accomplished by blood pressure monitoring system 10 and initiated by the application of power to electronics unit 20 of blood pressure monitoring system 10. After power has been applied to blood pressure monitoring system 10 a start routine illustrated in block 100 of Fig. 5A begins instruction execution. The address for this routine is fetched by microprocessor 44 at the reset exception vector and is stored as the initial program counter. The start routine then initializes the address registers and reverts program execution to the main portion of the program indicated in block 102. This portion of the program permits all hardware devices to be initialized as indicated in block 104 and the status register is cleared as indicated in block 106. Thereafter, a self test sequence is performed as indicated in block 108. The self test sequence consists of the same contents as a self test having blocks 116 to 138.

Referring to blocks 116 to 138, the diagnostic routine at block 108 will be described in brief. Among the tests indicated in the diagnostic mode of self test is the check ROM test indicated at block 118. The check ROM test is a cyclic redundancy check (CRC) as indicated in block 120 which is performed on the program ROM located at selected byte addresses within ROM 52. Additionally, a check RAM routine indicated by block 122 is performed and various RAM tests indicated by block 124 are performed on specific byte addresses within random access memory 54.

The diagnostic mode then tests the power supplies present within blood pressure monitoring system 10 and individual channels of multiplexer 78 are selected and verified for a specified range as indicated in block 126.

Data within individual channels of multiplexer 78 are then acquired as indicated in block 128 by selecting the desired analog signal and converting from analog to digital state.

Blood pressure monitoring system 10 next allows the operator to check the displays, alarm LEDS, switch LEDS and tone alarm for functionality during the check indicated in block 130. Validation of programmable parameters of the real time clock 58 is performed as indicated in blocks 132 and 134 and the cuff pressure transducer circuitry is tested as indicated in block 136. Dump valve 74 and bleed valve 72 are opened at the start of this self test in order for the transducer to stabilize prior to the test occuring. The cuff pressure channel on multiplexer 78 is selected and the output from the analog to digital converter 80 is read. This reading corresponds to a cuff pressure relatively close to zero. That is, the output signal corresponds to atmospheric pressure.

After the self test is complete, the blood pressure monitoring system 10 is placed into the stand-by mode as indicated in block 110.

Operation of blood pressure monitoring system 10 is then begun as indicated in block 112 by a routine which monitors the user's selection of front panel control keys and switches 66. Contents of the switch buffers is monitored each time the routine is called and the switch buffer contents are compared to switch buffer contents stored in a previous cycle to detect the user's inputs.

In the following step, in the event that the blood pressure monitoring system 10 has failed a self test, an error message is displayed.

The self test sequence takes approximately ten seconds to complete and results in displaying a diagnostic message when an error occurs during the self test. Additionally, tone alarm sounds for one second to indicate that an error message from the diagnostic mode has been displayed.

In the event that the self and diagnostic tests are not complete and that control panel position key 96 is not operated, program execution is repeated in block 112. In the event that position key 96 is detected to be operated, programing routine in block 114 is performed. This routine of block 114 consists of the same contents as blocks 139 to 146 (see Fig. 5), and monitors parameters corresponding to twelve positions of position key 96.

The self test may be performed again by pressing self test key 93 of switches 66 as indicated in block 116 and the following blocks. The twelve position parameters of position key 96 may be programmed by the programming routine as indicated in block 139 and the following blocks. Any changes in the switch position of position key 96 cause the switch parameter to be updated as indicated in block 144 and the new switch parameter thereafter stored per block 146.

Referring now to Figure 5B, additional logic flow chart diagrams are depicted which demonstrate the various operational modes of blood pressure monitoring system 10.

The manual mode routine indicated at block 148 provides for manual operation of blood pressure monitoring system 10. The manual mode of operation is entered by pressing manual key 92 on electronics unit 20. After calibrating the cuff pressure as indicated at block 150 and checking the system as for the power supplies as indicated in block 152, blood pressure monitoring system 10 will pump inflatable cuff 22 up to the pressure selected by the operator as indicated in block 154 and then begin bleeding inflatable cuff 22 at a rate of 3 mmHg per second as indicated in block 156. This method terminates when inflatable cuff 22 has been deflated to a pressure below 40 mmHg or until a set key (not shown) and a manual stop key (not shown) are depressed at one time.

Next, the rest mode may be utilized to obtain base line blood pressure measurements while patient 12 is at rest. Base line information from the rest mode is required to be prior to activating the exercise mode. To begin operation in the rest mode, the operator presses a stop/start push button (not shown) to select the run state which is indicated by the lightening of an LED. The preprocess function indicated at block 158 of Fig. 5C then occurs and a complex sequence of events takes place. The pressure transducer 76 is calibrated, as indicated in block 150, blood pressure monitor system 10's power supplies are tested as indicated in block 152 and all displays are reset to zero. The Abort "3" procedure is then utilized to determine based on signal to noise (S/N) ratio whether or not an electrocardiographic lead signal is present. If the Abort "3" flag indicates a loss of electrocardiographic lead signal, the Abort "3" routine, as indicated in block 160, is activated and an alternate electrocardiographic lead is selected as indicated in block 164 and if lead selection has been completed microprocessor 44 will then clear the status register SR as indicated in block 166 to begin operations for controlling interruptings and holding the results from calculations.

Block 168 is then utilized to calculate the interval in time of a measurement cycle by adding the current time and the interval time setting as established by the operator. As above, inflatable cuff 22 is then pumped up as indicated in block 154 until the pressure within inflatble cuff 22 reaches the desired cuff pressure.

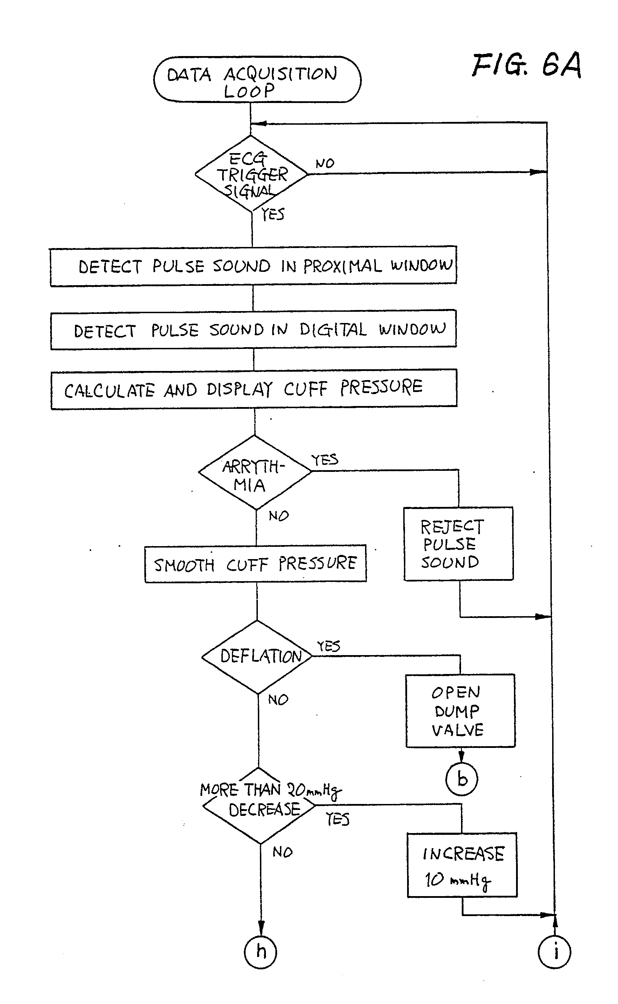

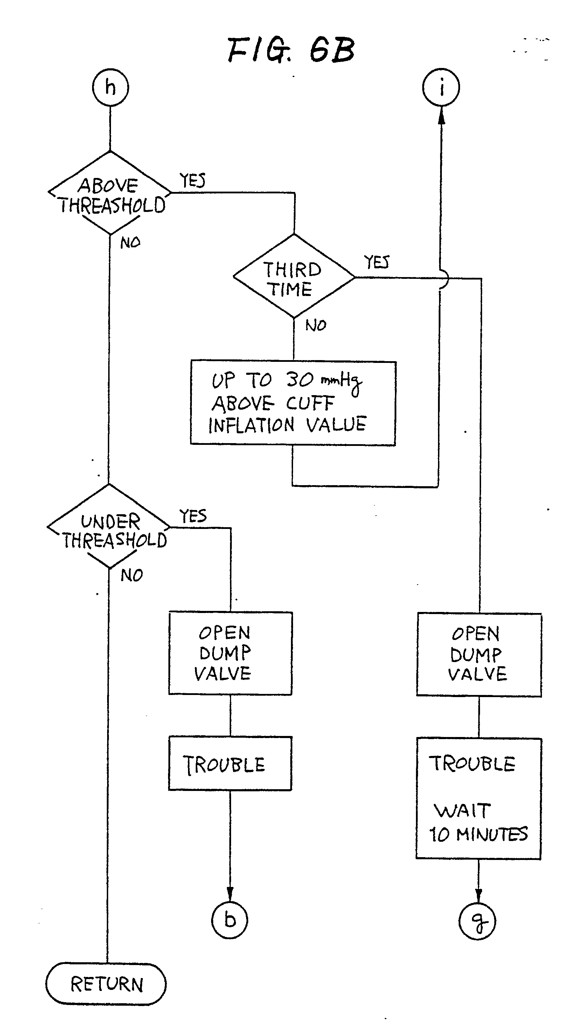

Referring now to Figures 6A and 6B, the data acquisition mode of blood pressure monitoring system 10 indicated by block 170 labeled "Process" has several steps in a loop that are performed while the cuff 22 is depressurizing. These steps include: (1) waiting for an electrocardiographic trigger to occur; (2) finding a pulse indicated by the proximal audio transducer output signal; (3) finding a pulse indicated by the distal audio transducer data; (4) finding the average cuff pressure for that pulse; (5) checking for an arrhythmia or false trigger condition; (6) performing a real time sort by cuff pressure of the data; (7) checking the cuff pressure against the value needed to open the dump valve and exit from data aquisition loop; (8) recovering from temporary loss of an electrocardiographic signal interrupt if necessary; (9) checking the amplitude of the distal pulses and reinflating the cuff if a large amplitude occurs near the beginning of the measurement; and (10) checking the amplitude of the proximal and stopping the measurement if the amplitude is too low.

The processing procedure utilized by blood pressure monitoring system 10 utilizes a concept of "windowing" in the description of the proximal and distal audio transducer data aquisition process. "Windowing" is the process utilized to reduce the amount of data that is examined for each pulse. Described in detail, in the windowing process, a pulse is monitored only for an "open" time during which the pulse is expected to occur, and as a result unnecessary noise that may be present in the "closed" time can be eliminated. The windowing process of blood pressure monitoring system 10 corresponds to gate means of the present invention. The size of the window, i.e., open time of the same is determined by previous data and its location is determined by a trigger from another event.

A pulse detected by the proximal audio transducer is searched for in a window of the raw proximal audio transducer data. The window begins 25 milliseconds after the occurrence of an electrocardiographic pulse and continues for 250 milliseconds during the first measurement. Thereafter, the duration is adjusted as discussed below. A subroutine (not shown) is utilized to find the location and peak-to-peak amplitude of the proximal pulse. When the proximal pulse has been located and its amplitude is stored in memory, the delay time from electrocardiographic trigger to proximal pulse is calculated by subtracting the location in time of the electrocardiographic trigger from the location in time of the proximal pulse in memory and then saving that data.

The distal pulse is searched for in a window of the raw distal audio transducer data. This window begins 50 milliseconds after the location of the proximal pulse that was just found. The distal window is open for 225 milliseconds. The same subroutine is then utilized to find the location and peak-to-peak amplitude of the distal pulse. The proximal pulse to distal pulse delay time is calculated by subtracting the location in time of the proximal pulse from the location in time of the distal pulse in memory. The delay time between these two pulses and pulse amplitude are then saved.

The next step in the processing loop is to find the cuff pressure for a given pulse. An average of the fifty cuff pressure points corresponding to the distal pulse window is calculated and saved. The calculated average cuff pressure is then displayed if it is less than the cuff pressure of the previous pulse. This check is made to prevent the display of temporary changes in cuff pressure. This condition is common during exercise measurements because arm movement can cause large temporary fluctuations in cuff pressure.

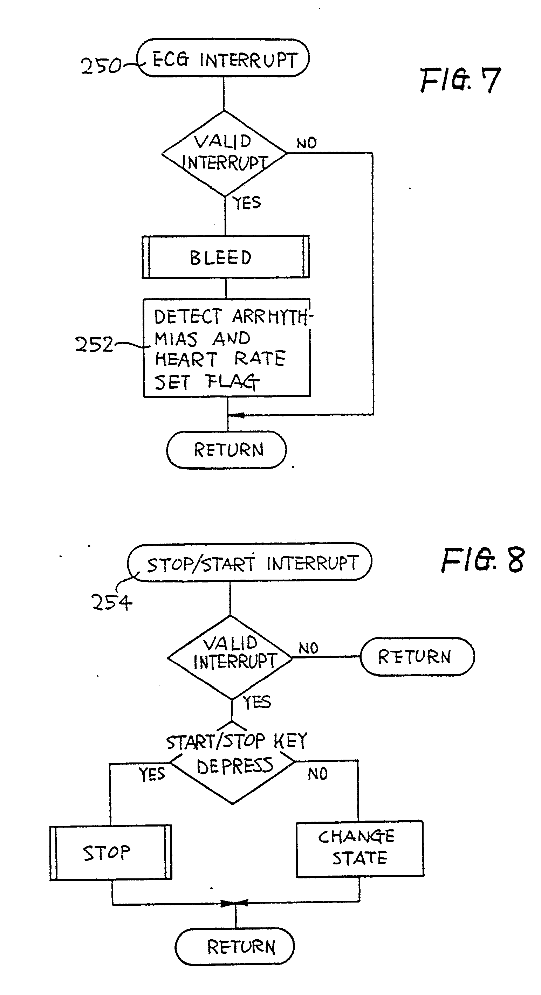

Referring now to Figure 7, the process program next checks for an arrhythmia condition by examining a flag set by the electrocardiographic interrupt handler. If the arrhythmia flag is set and the pulse amplitude is near the noise level and the cuff pressure is within the previous systolic to diastolic envelope, that pulse is rejected as a probable false trigger pulse.

The next step in the data aquisition loop is the real-time ordering of cuff pressure values. This technique is employed because of the possibility of a temporary cuff pressure increase due to arm movement. Ordering the cuff pressures allows for the maximum amount of data to be available for blood pressure detection. The technique begins by comparing the cuff pressure of the present pulse with the immediately preceding average cuff pressure array value. If the present cuff pressure is less, no action is needed; if the present cuff pressure is equal to the previous value, the present value is ignored. If the present value is greater than the previous value, the two cuff pressures swap positions in the array along with the corresponding pulse and delay data. The present value, in its new position, is then compared with its new, immediately preceding value and swapped if necessary.

Next, the present cuff pressure is compared with the predetermined value which is necessary to open dump valve 74. The predetermined value is held, for example, 40 mmHg which is less than a general diastlic blood pressure.

If dump valve 74's opening value has been reached, an external flag is set. An internal flag is also set that allows the data acquisition loop to terminate.

The next section of the data aquisition processing loop attempts to recover from a temporary loss of the electrocardiographic trigger signal. If a difference of more than 20 mmHg exists between the cuff pressure of the present and the previous pulses, then the cuff 22 is reinflated to a pressure 10 mmHg greater than the previous cuff pressure value. In this way, pulse data that should have been acquired during the 20 mmHg gap can be acquired.

Next, the amplitude of the output of the distal audio transducer 38 is then compared with the nominal distal pulse threshold for the first five pulses of the measurement. If the distal pulse amplitude is above this threshold, cuff pressure is probably insufficient to obtain distal data above the systolic level. If two consecutive distal pulses are above the threshold, the cuff 22 is reinflated to a point 30 mmHg above the initial inflation value. This reinflation may occur twice. If a third reinflation is necessary, the dump valve 74 is opened and a message is displayed to the operator. The machine then waits ten seconds and re-initiates the measurement procedure.

The amplitude of the proximal pulse is also compared with a noise threshold for the first eight pulses of the measurement. The amplitude of the proximal pulses should be significant near the beginning of the measurement. A low proximal amplitude could signal a faulty audio transducer 40 or poor positioning of the cuff 22. If three consecutive pulses are less than the threshold value, the measurement is aborted, and a message is displayed to the operator.

After the pulse data has been collected, the further processing of that data consists of several steps in addition to the actual calculation of blood pressure. Included among these steps are: (1) adjustment of the length of the proximal pulse window for the next cycle; (2) multiplying the proximal pulse to the distal pulse delay time and the corresponding distal pulse amplitude to obtain the product data set; (3) median filtering the distal amplitude data set and the product data set; (4) weighted average filtering the distal amplitude data set and the product data set; (5) determining blood pressure from both data sets and display the best results; (6) setting the cuff pump up value and the dump valve opening value for the next cycle; (7) calculation and display of the average heart rate; (8) calculation and display of the pulse rate product (PRP, systolic pressure X heart rate); (9) setting of maximum systolic and diastolic alarms if necessary; and (10) adjustment of the programmable attenuators for the next cycle.

As indicated at block 172 in Figure 5E, the proximal window length is readjusted during processing for the next cycle. This is advantageous because the time between the electrocardiographic trigger signal and the proximal pulse can vary widely from one cycle to the next. The dynamic quality of the proximal window length allows the minimum amount of raw data processing without sacrificing flexibility. The end of adjusted proximal window length is calculated by finding an average of the first ten electrocardiographic trigger signals to proximal pulse delay times and then adding 100 milliseconds to that average.

As indicated at block 173, the proximal to distal delay time is derived by subtracting the average electrocardiographic trigger signal to proximal pulse delay time from the electrocardiographic trigger signal to distal pulse delay time. The delay time is calculated in this way because the proximal pulse diminishes before the distal pulse, that is, the actual delay time is not reliable. Block 173 corresponds to time delay measurement means of the present invention.

Next, as indicated at block 174, the product data set is generated to determine the blood pressure evaluation curve. The product utilized is, as explained above, that of the distal amplitude and the proximal pulse to distal pulse delay time for that pulse.

The previously described product data set is the group of the thus obtained products. The product data set demonstrates the relation between the products and the cuff pressures, such as blood pressure evaluation curve shown in Figure 4C. The blood pressure evaluation curve is utilized to determine systolic and diastolic pressure. Block 174 corresponds to calculating means of the present invention.

The median filter and weighted average filter are then applied to both the amplitude data and the product data set as indicated in block 176. The primary function of the median filter is to remove single or double point artifacts from the data set. The median filter does this very well but it can also cause consecutive data points to be equal. This can cause the data set to have a stairstep appearance. The weighted average filter removes these stairsteps and further smooths the data. This filter produces a new output data point by adding a value to the previous input data point. This value is one-half the difference between the current and previous input data points.

The process of determining the systolic and diastolic pressures from each of the two mentioned data sets utilized by blood pressure monitoring system 10 begins with finding the value and position of the largest distal pulse in the data set as indicated in block 178. Block 178 corresponds to blood pressure measurement means of the present invention, in which large changings at which the evaluation curve rapidly increases or decreases are found so as to determine the systolic and diastolic blood pressure.

Another important feature of blood pressure monitoring system 10 of the present invention is that the cuff inflation value and dump valve opening value may be changed from one cycle to the next due to the fact that blood pressure may change greatly during a stress test. This is accomplished by adding a value to the systolic pressure to obtain the cuff inflation value to the next cycle and by subtracting a value from the diastolic pressure to obtain the dump valve opening value for the next cycle. An error resulting in a low systolic reading can cause successive measurements to be in error due to insufficient inflation of inflatable cuff 22. Blood pressure monitoring system 10 attempts to avoid this problem by comparing the present systolic pressure to the systolic pressure from the previous measurement. The over pump value is then added to the greater of the two pressures. This has the effect of not allowing a single poor measurement to affect the inflation value for the next measurement. If blood pressure monitoring system 10 is in the rest mode a value of 30 mmHg is added to the systolic pressure and a value of 20 mmHg is subtracted from the diastolic pressure. If blood pressure monitoring system 10 is in the exercise mode, a value of 50 mmHg is added to the systolic pressure and a value of 30 mmHg is subtracted from the diastolic pressure.

Furthermore, at block 179, the average heart rate is calculated based on electrocardiographic trigger signals, and the resultant value is displayed.

At the following block 180 of Fig. 5F, the pulse rate product (PRP) is calculated by means of multiplying the systolic blood pressure by the heart rate, and the resultant value is displayed. The PRP is utilized to monitor an actual stress being applied to patient 12 during physical activity.

In keeping with the dynamic nature of blood pressure monitoring system 10, both proximal and distal audio transducers 38, 40 are equipped with software programmable attenuators. This is necessary because the amplitude of the pressure pulse signals increases as heart rate and systolic pressure increase and the amplitude decreases as the patient 12 returns to normal after exercising. The maximum amplitude of the signal detected by the audio transducers 38 and 40 are utilized to adjust the attenuator setting at the end of cycle as indicated in blocks 182 and 184.

Referring now to Figure 7, various interrupt signals within blood pressure monitoring system 10 may be utilized to interrupt the process just described. The first such interrupt is the electrocardiographic interrupt indicated at block 250. This interrupt routine services the electrocardiographic leads and is active only during the rest or exercise mode of blood pressure monitoring system 10's operation. A valid electrocardiographic interrupt check is made each time the routine is called. Block 250 may be utilized for such as blocks 170 and 180. The electrocardiographic interrupt is used to keep a running time count of the last six electrocardiographic pulses to determine and display the average heart rate. This running time count includes arrhythmias and is updated every time after the first electrocardiographic trigger. The average heart rate value is visible on front panel 42 of electronic unit 20 and is updated every two seconds. Arrhythmia detection is done after the first three electrocardiographic trigger signals. Thereafter, a running three pulse time count is kept and compared to the previous running three pulse time count. An arrhythmia has occurred when the current pulse time deviates from the last pulse time by twenty-five percent. This process is indicated by block 252.

This routine includes a step of controlling the rate of deflation through bleed valve 72 each time the electrocardiographic trigger signal is generated.

A stop/start interrupt as indicated at block 254 of Figure 8 is utilized to change mode operation from stop to start or from start to stop each time the user depresses and releases the stop/start key. The start mode initiates the rest, exercise and manual modes of operation. The stop mode halts the activity of the current operation except for the self test and programming modes.

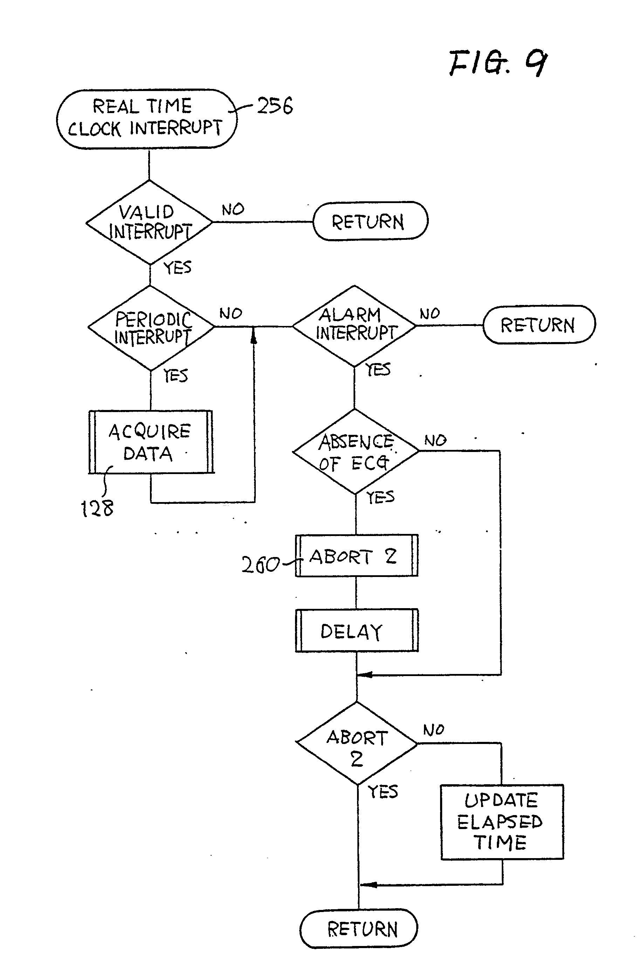

Finally, the real-time clock interrupt signal indicated at block 256 of Figure 9 services two of three possible sources of real-time clock interrupts to microprocessor 44, the alarm interrupt and the periodic interrupt. Alarm interrupts are initiated by the preprocess routine which activates a interrupt to update the elapsed time at the elapsed time display. The elasped time continues to update, but the display retains a user message indicating that the operator should check the system until the error condition is corrected. If an electrocardiographic trigger lead is detected as being detached, then the "Abort 2" subroutine indicated at block 260 is utilized to delay operation so that a proper electrocardiographic lead may be selected.

Finally, referring to Figure 5D, the process by which blood pressure monitoring system 10 selects the optimum electrocardiographic lead configuration is illustrated. The select lead routine indicated by block 262 is utilized to determine which of the three electrocardiographic leads is utilized as a trigger lead. Each of the electrocardiographic leads is analyzed as indicated in block 264 to determine which lead has the largest signal to noise (S/N) ratio. After a particular lead has been detected as having the largest signal to noise ratio, that lead is monitored and utilized to generate the electrocardiographic trigger signal. Each electrocardiographic lead may be periodically checked for commercial frequency noise present, and the lead having the least possible noise is selected. Thus, such leads as having weak lead signals and as having no lead signals due to detachment of the corresponding electrocardiographic electrode are avoided. The best lead is selected to generate the trigger lead signals. When an electrocardiographic electrode is detached from patient 12, the corresponding lead has no signal and has a large amount of commercial frequency noise. In this case, each of the above two select lead methods permits another normal electrode to be automatically selected.

While the present invention has been described in detail with reference to the preferred embodiment, it is appreciated that the invention may be modified with various changes and improvements without departing from the scope of the invention.

For example, in the above embodiment, the blood pressure evaluation curve is utilized to determine both systolic and diastolic pressure. The evaluation curve may be used to determine one of the two.

While the above embodiment is described for measurement on patient 12 during physical activity, it may be utilized for patient 12 when quiescent. In such case, the filtering processes, statistical processes, and windowing processes may not be used.

In the above embodiment, the statistical techniques are utilized to detect large changings on the evaluation curve, the predetermined reference threshold may be used to determine the blood pressure.

While the logic flow diagrams illustrated in Figures 5A, 5B and 5C are meant to illustrate the operation of blood pressure monitoring system 10, those ordinarily skilled in the art will appreciate that alternate algorithms may be utilized which will not depart substantially from the spirit and scope of this invention. Variations in the program may be suggested to those oridinarily skilled in the art upon reference to this specification and it is contemplated that the appended claims will cover these and other modifications or embodiments that fall within the true scope of this invention.

## Claims

1. A method of obtaining accurate blood pressure measurement by utilizing an inflatable cuff applied to a part of a subject's body, said method comprising the steps of:

   detecting a pulse sound at a proximal position of said inflatable cuff;

   detecting said pulse sound at a distal position of said inflatable cuff;

   measuring an amplitude of said pulse sound detected at the distal position of said inflatable cuff;

   measuring a cuff pressure in said inflatable cuff at the time of detecting said pulse sound at the distal position of said inflatable cuff;

   measuring a time delay between each pulse sound at the proximal position of said inflatable cuff and said pulse sound at the distal position of said inflatable cuff;

multiplying said amplitude of said pulse sound at the distal position of said inflatable cuff by said time delay; and

determining a measure of systolic and/or diastolic blood pressure of said body based on the relation between said resultant products and said cuff pressures.

2. A blood pressure monitoring system for measuring blood pressure by utilizing an inflatable cuff applied to a part of a subject's body, said blood pressure monitoring system comprising:

   proximal audio transducer means for detecting a pulse sound at a proximal position of said inflatable cuff;

   distal audio transducer means for detecting said pulse sound at a distal position of said inflatable cuff and for measuring an amplitude of said pulse sound detected;

   pressure transducer means for measuring a cuff pressure in said inflatable cuff at the time of detecting said pulse sound at the distal position of said inflatable cuff;

   time delay measurement means for obtaining a time delay between each pulse sound at the proximal position of said inflatable cuff and said pulse sound at the distal position of said inflatable cuff;

   calculating means for multiplying said amplitude of said pulse sound at the distal position of said inflatable cuff by said time delay; and

   blood pressure measurement means for obtaining a measure of systolic and/or diastolic blood pressure based on the relation between said resultant products and said cuff pressures.

3. A blood pressure monitoring system for measuring blood pressure by utilizing an inflatable cuff applied to a part of a subject's body, said blood pressure monitoring system comprising:

   proximal audio transducer means for detecting a pulse sound at a proximal position of said inflatable cuff;

   distal audio transducer means for detecting said pulse sound at a distal position of said inflatable cuff and for measuring an amplitude of said pulse sound detected;

   pressure transducer means for measuring a cuff pressure in said inflatable cuff at the time of detecting said pulse sound at the distal position of said inflatable cuff;

   electrocardiographic lead means providing at least one electrocardiographic lead which provides an electrocardiographic signal from said subject's body;

   gate means for permitting said pulse sound to pass therethrough for a predetermined period in response to said electrocardiographic signal;

time delay measurement means for obtaining a time delay between each pulse sound at the proximal position of said inflatable cuff and said pulse sound at the distal position of said inflatable cuff;

calculating means for multiplying said amplitude of said pulse sound at the distal position of said inflatable cuff by said time delay; and

blood pressure measurement means for obtaining a measure of systolic and/or diastolic blood pressure based on the relation between said resultant products and said cuff pressures.

4. A blood pressure monitoring system according to claim 3, wherein said electrocardiographic lead means comprises a plurality of electrocardiographic electrodes which are attached to said body, said plurality of electrocardiographic electrodes providing said at least one electrocardiographic lead.

5. A blood pressure monitoring system according to claim 3, wherein said at least one electrocardiographic lead comprises a plurality of electrocardiographic leads and wherein said electrocardiographic lead means selects a particular electrocardiographic lead from said plurality of electrocardiographic leads.

6. A blood pressure monitoring system according to claim 5, wherein said electrocardiographic lead means comprises means for periodically measuring the amount of noise present on each of said plurality of electrocardiographic leads, and also comprises means for selecting an electrocardiographic lead having the least amount of noise from said plurality of electrocardiographic leads.

7. A blood pressure monitoring system according to claim 5, wherein said electrocardiographic lead means comprises means for detecting the presence or absence of an electrocardiographic signal and wherein loss of said signal from said particular electrocardiographic lead will cause said electrocardiographic lead means to utilize a second lead.

## Revendications

1. Procédé de mesure précise de la pression sanguine grâce à l'utilisation d'une manchette gonflable appliqué sur une partie du corps du sujet, ledit procédé comprenant les étapes suivantes:

détection d'un son de pulsation en une position proximale de ladite manchette gonflable;

détection dudit son de pulsation en une position distale de ladite manchette gonflable;

mesure de l'amplitude dudit son de pulsation détecté sur la position distale de ladite manchette gonflable;

mesure de la pression de manchette, régnant dans ladite manchette gonflable au moment de la détection dudit son de pulsation, à la position distale de ladite manchette gonflable;

mesure de l'intervalle de retard entre chaque son de pulsation en position proximale de ladite manchette gonflable et ledit son de pulsation à la position distale de ladite manchette gonflable;

multiplication de ladite amplitude dudit son de pulsation en position distale de ladite manchette gonflable par ledit intervalle de retard; et

détermination de la mesure de la pression sanguine, systolique et/ou diastolique, dudit corps, sur la base de la relation entre lesdits produits résultants et lesdites pressions de manchette.

2. Système de surveillance de la pression sanguine, pour mesurer la pression sanguine grâce à l'utilisation d'une manchette gonflable appliquée sur une partie du corps humain, ledit système de surveillance de la pression sanguine comprenant:

un moyen transducteur audio proximal pour effectuer la détection d'un bruit de pulsation en une position proximale de ladite manchette gonflable;

un moyen transducteur audio distal pour effectuer la détection dudit son de pulsation en une position distale de ladite manchette gonflable;

un moyen transducteur de pression pour effectuer la mesure de la pression de manchette, régnant dans ladite manchette gonflable au moment de la détection dudit son de pulsation, à la position distale de ladite manchette gonflable;

un moyen de mesure d'intervalle de temps, pour effectuer la mesure de l'intervalle de retard entre chaque son de pulsation en position proximale de ladite manchette gonflable et ledit son de pulsation à la position distale de ladite manchette gonflable;

un moyen de calcul pour effectuer la multiplication de ladite amplitude dudit son de pulsation en position distale de ladite manchette gonflable par ledit intervalle de retard; et

un moyen de mesure de la pression sanguine, pour obtenir une mesure de la pression sanguine, systolique et/ou diastolique, sur la base de la relation entre lesdits produits résultants et lesdites pressions de manchette.

3. Système de surveillance de la pression sanguine, pour mesurer la pression sanguine grâce à l'utilisation d'une manchette gonflable appliquée sur une partie du corps humain, ledit système de surveillance de la pression sanguine comprenant:

un moyen transducteur audio proximal pour effectuer la détection d'un son de pulsation en une position proximale de ladite manchette gonflable;

un moyen transducteur audio distal pour effectuer la détection dudit son de pulsation en une position distale de ladite manchette gonflable;

un moyen transducteur de pression pour effectuer la mesure de la pression de manchette, régnant dans ladite manchette gonflable au moment de la détection dudit son de pulsation, à la position distale de ladite manchette gonflable;

un moyen conducteur électrocardiographique fournissant au moins un conducteur électrocardiographique qui envoie un signal électrocardiographique provenant dudit corps humain;

un moyen de porte logique, pour permettre audit son de pulsation de passer à travers pendant une période prédéterminée, en réponse audit signal électrocardiographique;

un moyen de mesure d'intervalle de temps, pour effectuer la mesure de l'intervalle de retard entre chaque son de pulsation en position proximale de ladite manchette gonflable et ledit son de pulsation à la position distale de ladite manchette gonflable;

un moyen de calcul pour effectuer la multiplication de ladite amplitude dudit son de pulsation en position distale de ladite manchette gonflable par ledit intervalle de retard; et

un moyen de mesure de la pression sanguine, pour obtenir une mesure de la pression sanguine, systolique et/ou diastolique, sur la base de la relation entre lesdits produits résultants et lesdites pressions de manchette.

4. Système de surveillance de la pression sanguine selon la revendication 3, dans lequel ledit moyen conducteur électrocardiographique comprend une pluralité d'électrodes électrocardiographiques fixées sur ledit corps, ladite pluralité d'électrodes électrocardiographiques fournissant au moins un conducteur électrocardiographique.

5. Système de surveillance de la pression sanguine sel on la revendication 3, dans lequel ledit au moins un moyen conducteur électrocardiographique comprend une pluralité de conducteurs électrocardiographiques et dans lequel ledit moyen conducteur électrocardiographique sélectionne un conducteur électrocardiographique particulier parmi ladite pluralité de conducteurs électrocardiographiques.

6. Système de surveillance de la pression sanguine selon la revendication 5, dans lequel ledit moyen conducteur électrocardiographique comprend un moyen pour mesurer de façon périodique la valeur du bruit existant sur chacun parmi ladite pluralité de conducteurs électrocardiographiques, et comprend également un moyen pour sélectionner un conducteur électrocardiographique manifestant le plus faible bruit, parmi la pluralité de conducteurs électrocardiographiques.

7. Système de surveillance de la pression sanguine selon la revendication 5, dans lequel ledit moyen conducteur électrocardiographique comprend un moyen pour détecter la présence ou l'absence d'un signal électrocardiographique et dans lequel la perte dudit signal dans ledit conducteur électrocardiographique particulier provoque le passage, dans ledit moyen conducteur électrocardiographique, à l'utilisation d'un second conducteur.

## Ansprüche

1. Verfahren zum Erzielen einer genauen Blutdruckmessung mittels einer an einen Körperteil eines Patienten angelegten aufblasbaren Manschette, mit den Schritten:

Erfassen eines Pulstons an einer körpernahen Stelle der aufblasbaren Manschette,

Erfassen des Pulstons an einer körperfernen Stelle der aufblasbaren Manschette,

Messen einer Amplitude des an der körperfernen Stelle der aufblasbaren Manschette erfassten Pulstons,

Messen eines Manschettendrucks in der aufblasbaren Manschette zum Zeitpunkt des Erfassens des Pulstons an der körperfernen Stelle der aufblasbaren Manschette,

Messen einer Verzögerungszeit zwischen dem jeweiligen Pulston an der körpernahen Stelle der aufblasbaren Manschette und dem Pulston an der körperfernen Stelle der aufblasbaren Manschette,

Multiplizieren der Amplitude des Pulstons an der körperfernen Stelle der aufblasbaren Manschette mit der Verzögerungszeit und

Ermitteln eines Masses für den systolischen und/oder diastolischen Blutdruck des Körpers aus dem Zusammenhang zwischen den sich ergebenden Produkten und den Manschettendrücken.

2. Blutdrucküberwachungssystem für das Messen von Blutdruck mittels einer an einen Körperteil eines Patienten angelegten aufblasbaren Manschette, enthaltend:

eine körpernahe Tonwandlervorrichtung für das Erfassen eines Pulstons an einer körpernahen Stelle der aufblasbaren Manschette,

eine körperferne Tonwandlervorrichtung für das Erfassen des Pulstons an einer körperfernen Stelle der aufblasbaren Manschette und für das Messen einer Amplitude des erfassten Pulstons

eine Druckwandlervorrichtung für das Messen eines Manschettendrucks in der aufblasbaren Manschette zum Zeitpunkt des Erfassens des Pulstons an der körperfernen Stelle der aufblasbaren Manschette,

eine Verzögerungszeitmeßeinrichtung für das Ermitteln einer Verzögerungzeit zwischen einem jeweiligen Pulston an der körpernahen Stelle der aufblasbaren Manschette und dem Pulston an der körperfernen Stelle der aufblasbaren Manschette,

eine Recheneinrichtung für das Multiplizieren der Amplitude des Pulstons an der körperfernen Stelle der aufblasbaren Manschette mit der Verzögerungszeit und

eine Blutdruckmeßeinrichtung für das Ermitteln eines Maßes für den systolischen und/oder diastolischen Blutdruck aus dem Zusammenhang zwischen den sich ergebenden Produkten und den Manschettendrücken.

3. Blutdrucküberwachungssystem für das Messen von Blutdruck mittels einer an einen Körperteil eines Patienten angelegten aufblasbaren Manschette, enthaltend:

eine körpernahe Tonwandlervorrichtung für das Erfassen eines Pulstons an einer körpernahen Stelle der aufblasbaren Manschette,

eine körperferne Tonwandlervorrichtung für das Erfassen des Pulstons an einer körperfernen Stelle der aufblasbaren Manschette und für das Messen einer Amplitude des erfassten Pulstons,

eine Druckwandlervorrichtung für das Messen eines Manschettendrucks in der aufblasbaren Manschette zum Zeitpunkt des Erfassens des Pulstons an der körperfernen Stelle der aufblasbaren Manschette,

eine EKG-Anschlußvorrichtung mit mindestens einem EKG-Kabel, das ein EKG-Signal vom Körper des Patienten liefert,

eine Torvorrichtung für das Durchlassen des Pulstons in einem vorbestimmten Zeitabschnitt im Ansprechen auf das EKG-Signal,

eine Verzögerungszeitmeßeinrichtung für das Ermitteln einer Verzögerungzeit zwischen einem jeweiligen Pulston an der körpernahen Stelle der aufblasbaren Manschette und dem Pulston an der körperfernen Stelle der aufblasbaren Manschette,

eine Recheneinrichtung für das Muliplizieren der Amplitude des Pulstons an der körperfernen Stelle der aufblasbaren Manschette mit der Verzögerungszeit und

eine Blutdruckmeßeinrichtung für das Ermitteln eines Masses für den systolischen und/oder den diastolischen Blutdruck aus dem Zusammenhang zwischen den sich ergebenden Produkten und den Manschettendrücken.

4. Blutdrucküberwachungssystem nach Anspruch 3, in dem die EKG-Anschlußvorrichtung eine Vielzahl von an dem Körper angebrachten EKG-Elektroden aufweist, die das mindestens eine EKG-Kabel bilden.

5. Blutdrucküberwachungssystem nach Anspruch 3, in dem das mindestens eine EKG-Kabel eine Vielzahl von EKG-Leitungen enthält und in dem die EKG-Anschlußvorrichtung aus der Vielzahl der EKG-Leitungen eine bestimmte EKG-Leitung wählt.

6. Blutdrucküberwachungssystem nach Anspruch 5, in dem die EKG-Anschlußvorrichtung eine Einrichtung für das periodische Messen des Ausmasses von an jeder der Vielzahl von EKG-Leitungen anliegenden Störsignalen und auch eine Einrichtung für das Wählen einer EKG-Leitung mit dem geringsten Ausmaß an Störsignalen aus der Vielzahl der EKG-Leitungen enthält.

7. Blutdrucküberwachungssystem nach Anspruch 5, in dem die EKG-Anschlußvorrichtung eine Einrichtung für das Erfassen des Vorliegens oder Fehlens eines EKG-Signals enthält und in dem der Ausfall des Signals aus der bestimmten EKG-Leitung bewirkt, daß die EKG-Anschlußvorrichtung eine zweite Leitung einsetzt.

# FIG. 1

# FIG. 2

FIG. 3

FIG. 4A

FIG. 4B

FIG. 4C

FIG. 4D

F/G.5

# FIG. 5A

# FIG. 5B

FIG. 5C

# FIG. 5D

(a") → ◇ AUTO LEAD SELECT ◇ — NO

YES

262 — | SELECT LEAD |

264 — | SEARCH MAX |

266 — | MONITOR LEAD |

| SET SR |

(f)

(b)

# FIG. 5E

(d)

| CALIBRATE PROX-IMAL WINDOW | — 172

| CALCULATE TIME DELAY | — 173

| PRODUCT DATA. | — 174

| FILTERING | — 176

| CALCULATE AND DISPLAY BLOOD PRESSURE | — 178

| DETERMINE CUFF INFLATION VALUE AND DUMP VALVE OPENING VALUE FOR NEXT MEASURE-MENT<br><br>CALCULATE AND DISPLAY AVERAGE HEART RATE | — 179

(d')

# FIG. 5F

(d')

| CALCULATE AND DISPLAY PRP | — 180 |

| ADJUST ATTENUATOR | — 182 |

| SET ATTENUATOR | — 184 |

BEGIN NEXT MEASUREMENT — YES → REST/EXER/ STOP — YES → (f)

NO (from BEGIN NEXT MEASUREMENT)

NO (from REST/EXER/STOP) → (g)

NO ← AUTO LEAD SELECT

YES

| SELECT LEAD | — 262 |

| SEARCH MAX | — 264 |

| MONITOR LEAD | — 266 |

# FIG. 6A

# FIG. 6B

h

ABOVE THREASHOLD — YES / NO

THIRD TIME — YES / NO

UP TO 30 mmHg ABOVE CUFF INFLATION VALUE

i

UNDER THREASHOLD — YES / NO

OPEN DUMP VALVE

TROUBLE

b

OPEN DUMP VALVE

TROUBLE

WAIT 10 MINUTES

g

RETURN

250 — ( ECG INTERRUPT )

**FIG. 7**

VALID INTERRUPT — NO

YES

BLEED

252 — DETECT ARRHYTH-MIAS AND HEART RATE SET FLAG

( RETURN )

254 — ( STOP/START INTERRUPT )

**FIG. 8**

VALID INTERRUPT — NO — ( RETURN )

YES

START/STOP KEY DEPRESS

YES

NO

STOP

CHANGE STATE

( RETURN )

FIG. 9